(19) **Europäisches Patentamt**

**European Patent Office**

**Office européen des brevets**

(11) Publication number: **0 553 251 B1**

(12) **EUROPEAN PATENT SPECIFICATION**

(45) Date of publication of patent specification: **18.01.95**

(51) Int. Cl.$^6$: **C07C 53/126**, C07C 53/128, C07C 51/41

(21) Application number: **91919687.3**

(22) Date of filing: **18.10.91**

(86) International application number:
**PCT/EP91/02009**

(87) International publication number:
**WO 92/06945 (30.04.92 92/10)**

(54) **METAL CARBOXYLATES.**

(30) Priority: **19.10.90 GB 9022805**

(43) Date of publication of application:
**04.08.93 Bulletin 93/31**

(45) Publication of the grant of the patent:
**18.01.95 Bulletin 95/03**

(84) Designated Contracting States:
**AT BE CH DE ES FR GB GR IT LI LU NL**

(56) References cited:
**EP-A- 0 010 807**
**EP-A- 0 279 493**
**FR-A- 1 540 384**
**GB-A- 1 005 957**
**GB-A- 8 918 58**

(73) Proprietor: **EXXON CHEMICAL PATENTS INC.**
**200 Park Avenue**
**Florham Park**
**New Jersey 07932 (US)**

(72) Inventor: **SARGINSON, Nigel, James**
**Constantin Meunierlaan 1**
**B-3090 Overijse (BE)**
Inventor: **GODWIN, Allen, David, 335 Bukit**
**Timah Road**
**07-03 Wing on Life**
**Singapore 1025 (SG)**
Inventor: **REYNIERS, Sylvain, Leontina, Edmond**
**Peter Benoitlaan 2**
**B-1880 Kapelle-op-den-Bos (BE)**

(74) Representative: **Bawden, Peter Charles et al**
**EXXON CHEMICAL LIMITED**
**EXXON CHEMICAL TECHNOLOGY CENTRE**
**PO Box 1**
**Abingdon**
**Oxfordshire OX13 6BB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

The invention relates to basic barium carboxylates, to a process for the preparation of such carboxylates, and to the use of the carboxylates for, inter alia, stabilizing halogen-containing organic polymers.

Many halogen-containing organic polymers, including, for example, polyvinyl chloride (PVC), must be protected against degradation and discoloration if they ore to be exposed to high temperatures or strong light for any length of time. At the elevated temperatures which are necessary for thermoplastic processing, PVC is damaged by dehydrochlorination (release of hydrogen chloride), autoxidation and mechanochemical chain scission. Dehydrochlorination is the main reaction during thermal processing and leads to increasing discoloration of the PVC. A major function of PVC stabilizers is binding and neutralization of the released hydrogen chloride. Other functions include substitution of labile chlorine atoms, additions to polyene sequences, destruction of carbenium salts, and prevention of antoxidation.

A variety of stabilizers have been proposed for halogen-containing polymers, including, for example, the basic alkali and alkaline earth metal salts disclosed in U.S. Specification No. 4 665 117 and the barium- and cadmium-containing organic complexes disclosed in U.S. Specification No. 3 194 823. U.S. Specification No. 4 665 117 is concerned with a process for improving the colour and stability of basic alkali and alkaline earth metal salts prepared from mixtures containing a phenol. In one embodiment of the process, the reaction mixture contains at least one monocarboxylic acid as well as at least one phenol. The monocarboxylic acid may be an aliphatic or aromatic acid, the aliphatic acids generally having 6 to 30 carbon atoms, with the higher fatty acids containing an average of at least about 12 carbon atoms being preferred. The basic complexes disclosed in U.S. Specification No. 3 194 823 contain cadmium as well as barium. The specification states that the excess metal is believed to be present as colloidally dispersed metallic carbonate, but that otherwise little is known about the chemical composition of the complexes.

Despite the disclosures of the above-numbered specifications, there remains a need for stabilizers which are easy to prepare, which can readily be dispersed in polymeric materials to be stabilized, and which are highly effective stabilizers for those materials.

The present invention provides a basic barium carboxylate wherein at least 85% weight of the carboxylate groups consist of groups derivable from at least one branched chain aliphatic monocarboxylic acid having 6 to 9 carbon atoms, preferably 7 to 9 carbon atoms.

The carboxylates of the invention may contain minor amounts of carboxylate groups derived from acids which are not branched chain aliphatic C6 to C9 monocarboxylic acids. Preferably no more than 10 wt %, and especially not more than 5 wt %, of the carboxylate groups, based on the total weight of the acids from which the carboxylate groups are derived, are derived from acids other than the specified C6 to C9 acids.

A basic barium carboxylate of the invention may be derived from one, substantially pure, branched chain aliphatic monocarboxylic acid having 6 to 9 carbon atoms. Because commercial processes (e.g., the oxo process carried out on hydrocarbon oligomer feedstocks) for the preparation of such acids normally result in a mixture of products, however, the basic barium carboxylates will more normally be derived from a mixture consisting essentially of two or more branched chain aliphatic monocarboxylic acids having 6 to 9 carbon atoms. Where at least two branched chain aliphatic C6 to C9 monocarboxylic acids are present, a first such acid may contain the same number of carbon atoms as a or the second, but be in a different isomeric form from the second, or a first such acid may contain a different number of carbon atoms from a or the second. A mixture of acids containing two or more isomers having a particular number of carbon atoms and at least one acid having a different number of carbon atoms may, of course, also be used.

The aliphatic chain, including the branching group(s), in the acids from which the basic barium carboxylates of the invention are derivable may contain, or be substituted by, one or more atoms or groups which are inert under the conditions to which the acids and carboxylates are subjected during processing and use, but, advantageously, the aliphatic chain, including the branching group(s), is made up of carbon and hydrogen atoms only. Furthermore, the aliphatic chain, including the branching group(s), is preferably saturated, as a basic barium carboxylate derived from an unsaturated acid could in some circumstances lead to discoloration of a polymer with which it is blended.

The acids from which the basic barium carboxylates are derivable may contain one or more branching groups, which are normally ethyl or methyl groups, on the main aliphatic chain. The branching group(s) may be present at any position on the main chain, although acids containing at least one branching group on a carbon atom other than the $\alpha$-carbon atom (the carbon atom adjacent to the carboxyl group) are preferred. For some purposes it is advantageous to have $\alpha$-branching in addition to non-$\alpha$-branching. Thus, for example, acids which are di-$\alpha$-branched as well as being non-$\alpha$-branched may give carboxylates with a reduced tendency to "plate-out" of polymers with which they are blended, that is, with a reduced tendency to migrate to the surface of the polymers during processing.

Examples of acids from which the basic barium carboxylates of the invention are derivable are the aliphatic C8 and C9 acids sold by Exxon Chemical under the trade name "Cekanoic", and the branched C7 and C9 neo acids sold by Exxon Chemical, the neo acids being di-α branched and also having branching elsewhere in the chain. Cekanoic C8 acid comprises predominantly C8 non-α branched isomers, while Cekanoic C9 acid is predominantly 3,5,5-trimethyl hexanoic acid. Other acids which may be used include the mixture of C9 isomers known as isononanoic acid, and neooctanoic acid, the last-mentioned acid having two substituents on the α-carbon atom and also being substituted elsewhere on the chain.

Mixtures of one or more of the acids, or types of acids, referred to above may of course be used.

The barium carboxylates of the invention are basic, or overbased, that is to say, the quantity of metal present is greater than that predicted by stoichiometry as being required to react completely with the monocarboxylic acid(s). The extent to which a salt is basic or overbased can be expressed in a number of different ways, but in this specification will be expressed as the basicity, that is, the ratio of metal equivalents to carboxylic acid equivalents. A neutral salt will have a basicity of 1, while a basic or overbased salt will have a basicity of greater than 1. A high basicity is advantageous as this minimizes the weight of any given carboxylic acid (and thus the weight of barium salt) required for the introduction of a specified weight of barium into, for example, a polymer to be stabilized. The basic carboxylates of the invention have been prepared with basicities of 2 or more, for example, up to about 3.

As will be explained in greater detail below, the preparation of the basic barium carboxylates normally involves, inter alia, the reaction of a barium compound with an acidic gas, that is, a gas which upon reaction with water will produce an acid, the preferred gas being carbon dioxide, the reaction taking place in an organic liquid in the presence of (a) the monocarboxylic acid(s) and a barium compound capable of reacting therewith and/or (b) a barium carboxylate obtainable from the acid(s) and barium compound specified in (a). The precise structure of the basic carboxylates is unknown, but it is thought to be micellar, the micelles, which comprise the barium salt of the acidic gas (for example, barium carbonate) surrounded by barium carboxylate molecules, being in the form of a colloidal solution in the organic liquid. When the carboxylate is used as a stabilizer for a polymer, it is this colloidal solution that is incorporated into the polymer, and the physical properties of the colloidal solution are thus of considerable importance.

The Applicants have surprisingly found that colloidal solutions of the basic barium carboxylates of the invention have, in a given organic medium and for a given barium content, significantly lower viscosities than colloidal solutions of neutral barium salts of C6 to C9 branched chain monocarboxylic acids and colloidal solutions of barium carboxylates having a greater number of carbon atoms. The viscosities are also significantly lower than those of certain other overbased barium salts previously used as stabilizers for polyvinyl chloride, for example, an overbased barium nonylphenolate containing 27.5 wt % barium. The relatively low viscosities of colloidal solutions of the carboxylates of the invention give rise to a number of advantages, including a saving in production costs because filtration, which is frequently a problem with overbased salts, is easier and quicker, better mixing and processability when the carboxylates are used in stabilizer formulations, including improved filterability if the formulations are to be filtered, and improved dispersion in a polymer, for example, polyvinyl chloride, to be stabilized.

The reduction in viscosities obtainable in accordance with the invention is illustrated in Table I below, where the basicity and viscosity of colloidal solutions in dearomatized white spirit of a number of different barium salts are given. In the table, the barium content of each colloidal solution is given as a percentage by weight, based on the weight of the colloidal solution.

TABLE I

| Acid from which barium salt derived | Barium Content wt% | Basicity | Viscosity at 20 °C $10^{-6}\,m^2/s$ |
|---|---|---|---|
| Cekanoic C8 | 13 | 1 | 441 |
| 2-Ethylhexanoic | 12.5 | 1 | 327 |
| Cekanoic C8 | 16 | 2 | 3.5* |
| Neodecanoic | 16 | 2 | 17.4 |
| Cekanoic C8 | 20 | 2.5 | 4.9* |
| Neodecanoic | 20 | 2 | 36 |
| Oleic | 34 | >1 | 332 (at 40 °C) |

*carboxylate according to the invention.

By way of further comparison, an overbased barium nonyl phenolate having a barium content of 27.5 wt % had a viscosity of $2300 \times 10^{-6}$ $m^2/s$ at 25°C.

It will be seen from Table I that the viscosities of the colloidal solutions of the overbased salts are substantially lower, by a factor of about 10 to 100, than those of neutral salts of similar acids, and, for the overbased salts, the carboxylates according to the invention have lower viscosities, for a given barium content, than the carboxylates of the closely similar C10 branched acid, neodecanoic acid. As discussed elsewhere in this specification, a further advantage of the salts of the invention over the corresponding overbased salts of branched C10 acids is the fact that the salts of the invention having a high barium content can be obtained directly, without the need for a concentration step, and higher basicities are in some cases obtainable with the acids used in accordance with the invention.

As well as the advantages arising from their low viscosities and high barium contents, the basic salts of the invention also have the advantage that they may have low colour, which is particularly significant where white or light-coloured polymers are to be stabilized.

Any suitable process may be used for preparing the basic barium carboxylates of the invention. As indicated above, such a process will normally involve the reaction of a basic barium compound with an acidic gas, especially carbon dioxide, in an organic medium containing (a) the monocarboxylic acid(s) and a barium compound capable of reacting with the acid(s) and/or (b) a barium carboxylate obtainable by the reaction of the acid(s) and barium compound specified in (a), and the invention accordingly also provides such a process. A promoter, preferably a hydroxylic group-containing promoter, for example, a phenol or, especially, an alcohol, is advantageously also present in the reaction medium.

Processes of the above general type are well known and are described in, for example, the above-mentioned U.S. Specifications Nos. 3 194 823 and 4 665 117, the disclosures of which are incorporated by reference herein. In view of this, it is believed unnecessary to discuss the process of the invention in detail, although process features that have been found to be particularly preferred when preparing the basic barium carboxylates of the invention are discussed briefly below.

Advantageously, the process involves the use of a single barium compound which is capable of reacting with the monocarboxylic acid(s) and with the acidic gas. Suitable barium compounds include barium oxide, barium sulphide, and barium hydrosulphide, but the preferred barium compound is barium hydroxide, which is advantageously used in the form of the octahydrate. Preferably, the reaction medium contains sufficient water to dissolve the barium compound, for example, the barium hydroxide octahydrate.

Preferred promoters for use in preparing the basic barium carboxylates of the invention are polyhydric alcohols, for example, glycols. Triethylene glycol is particularly preferred. A further promoter, for example, a phenol, may also be present, but is not essential. Further, methanol, used as a promoter in many prior overbasing processes, is not required, although its use is not excluded. The promoter is advantageously added to a mixture of the barium compound, water, and an organic liquid before introduction of the monocarboxylic acid. The amount of promoter used is not critical, but is advantageously in the range of from 3 to 15 wt%, based on the final colloidal solution.

Any organic liquid which is inert under the conditions used in the process may be used as the medium in which to carry out the reaction. As the overbased carboxylates are used without removal of the organic liquid, the choice of liquid will be dependent in part on the intended end use of the product. Preferably, a hydrocarbon liquid is used, for example, dearomatized white spirit, although an oxygen-containing liquid compound may be appropriate in some circumstances.

The branched aliphatic C6 to C9 monocarboxylic acid(s) used in accordance with the invention is/are advantageously introduced gradually into a mixture comprising the basic barium compound, the promoter, the organic liquid, and optionally water, whereupon the barium compound and acid(s) are thought to react to form the corresponding barium carboxylate(s). (If desired, barium carboxylate(s) could be introduced as such into the reaction medium, rather than being prepared in the medium, but it is normally more convenient to prepare the carboxylates in situ.) It is preferred that the mixture to which the acid is added be at elevated temperature, preferably in the range of 80 to 120°C.

The reaction medium into which the acidic gas is passed contains an amount of basic barium compound in excess of that required to react with the monocarboxylic acid(s). The acidic gas may be, for example, sulphur dioxide, sulphur trioxide, carbon disulphide or hydrogen sulphide, but is advantageously carbon dioxide. For convenience, the invention will hereafter be discussed with reference to carbon dioxide, but it will be appreciated that, where appropriate, this may be replaced by another acidic gas. Additional barium compound may, if desired, be added during carbonation, but preferably all the barium compound is introduced into the reaction vessel at the outset.

Advantageously, water present in the reaction medium is substantially completely eliminated, for example, by heating, before carbonation is commenced. Carbon dioxide is preferably passed through the reaction medium until no more is absorbed by the reaction medium, carbonation advantageously being effected at an elevated temperature, preferably a temperature of from 130 to 170°C, more preferably 140 to 170°C. The product obtained may then be filtered to remove any unreacted solids.

One or more steps to reduce any tendency to discoloration of the basic salts are advantageously included in the process. Thus, the process is preferably carried out, at least when carbonation is not occurring, under an atmosphere of an inert gas, for example, nitrogen. Further, an agent to improve the stability of the colloid, for example, an agent that will react with any free hydroxyl groups, is advantageously added to the final product. Preferred agents for stabilizing the colloid are alkyl phosphates, for example,triethyl phosphate.

Normally barium will be the only metal present in the carboxylates of the invention. For some purposes it may be desirable that zinc be present in addition to barium in, for example, a polymer to be stabilized, and, where this is the case, a barium carboxylate may be mixed with a zinc salt before incorporation into the polymer, or a zinc salt may be blended into the polymer separately. In some circumstances, however, it may be advantageous to incorporate zinc into the barium salts of the invention, for example, by replacing some of the barium compound(s) used as starting material by a zinc compound that will react with the carboxylic acid(s) in the reaction mixture used for preparing the overbased barium carboxylates, and the invention accordingly also provides a basic barium carboxylate according to the invention in the form of a complex which also contains zinc. For some purposes another metal, other than cadmium, may replace the zinc in the mixtures and complexes referred to above.

In accordance with the invention, it is possible, especially when the preferred process features referred to above are used, to obtain colloidal solutions of basic barium salts of C6 to C9 branched chain aliphatic monocarboxylic acids with a relatively high barium content (for example, 20 wt% or more) directly, without the use of a final concentration step to reduce the amount of organic liquid present. As indicated earlier, if the acid(s) used in accordance with the invention are replaced in whole or in substantial amounts by a higher acid, for example, a branched chain C10 aliphatic monocarboxylic acid, such barium contents can only be obtained by a process of the type described if a final concentration step is used.

As indicated above, the basic barium carboxylates of the invention may be used for, inter alia, reducing degradation and/or discoloration of halogen-containing polymers, for example, vinyl chloride polymers, which are exposed to high temperatures and/or light, for any length of time. Accordingly, the invention also provides a polymer composition which comprises a halogen-containing organic polymer, especially a vinyl halide polymer, and a basic barium salt in accordance with the invention. The invention also provides the use of a basic barium carboxylate in accordance with the invention for stabilizing a halogen-containing organic polymer, especially a vinyl halide polymer. The barium carboxylates of the invention are especially useful for stabilizing homopolymers and copolymers of vinyl chloride, particularly flexible vinyl chloride polymers.

In general, the basic salts are added to the polymer in such an amount that the proportion of barium in the composition is in the range of from about 0.01 to 0.5 wt % barium, preferably 0.025 to 0.25 wt % barium, based on the weight of the polymer and any other components, including plasticizer, that may be present.

Because the use of the specified C6 to C9 acids in the production of overbased barium salts makes it possible to produce salts having a higher proportion of barium than do the corresponding neutral salts, the same barium content can be obtained in a given weight of polymer composition using a lower weight of barium-containing additive than that necessary when using neutral salts. This makes it possible, if desired, to include other components in a stabilizer formulation to be added to a polymer. The invention therefore also provides a stabilizer formulation comprising a basic barium carboxylate according to the invention and a further component, for example, a co-stabilizer. Suitable co-stabilizers include phosphites, for example, diphenyl isooctyl phosphite. Such a formulation may contain, for example, 2 to 15 wt % of barium.

The basic barium carboxylates of the invention may also find application as paint dryers, corrosion inhibitors, and as detergent/dispersant additives for lubricating oils. Where desirable for any particular application, they may, as indicated above, be used in conjunction with other overbased metal salts. Thus, for example, zinc salts may be desirable constituents of stabilizers for PVC.

Examples 1 to 4 illustrate the invention. Examples A and B are provided by way of comparison.

## Comparative Example A

Preparation of a basic barium salt of neodecanoic acid.

A reactor was charged with 398 g of barium hydroxide octahydrate and 500 g of water and the mixture was heated slowly to 80°C with thorough stirring. 80 g of triethylene glycol and 327 g of dearomatized white spirit (two-thirds of the total quantity used) were then added, and the resulting mixture was heated slowly to 90 to 95°C. A mixture of 215 g of neodecanoic acid and 164 g of dearomatized white spirit were then slowly added over a period of 20 to 30 minutes, following which the mixture was slowly heated to approximately 105°C and maintained at that temperature for about 1 1/4 hours. Thereafter, the mixture was heated slowly to approximately 155°C in order to eliminate any remaining water.

After allowing the mixture to cool to about 140°C, it was carbonated for 25 minutes at a carbon dioxide flow rate of 48 litres/hour. The carbonated mixture was then allowed to cool to 120°C and filtered using a CUNO 308 Zeta Plus filter. The filtered product had a barium content of about 17 wt%, a basicity of 2, and a viscosity at 20°C of approximately $15 \times 10^{-6}$ $m^2/s$. This product was then concentrated by distillation under vacuum to give a product with a barium content of 20 wt % and a viscosity at 20°C of approximately $36 \times 10^{-6}$ $m^2/s$. 2 wt % of triethylphosphate, based on the weight of the concentrated product, was then added to improve the stability of the colloidal solution.

All the steps indicated above, apart from carbonation, were carried out under an atmosphere of nitrogen.

## Comparative Example B

Preparation of a basic barium salt of a mixture of neodecanoic acid and neononanoic acid.

The procedure indicated in Comparative Example A was repeated, replacing the neodecanoic acid by 217 g of a mixture of neodecanoic acid and neononanoic acid (70 parts by weight of C10 acid to 30 parts by weight of C9 acid). A product containing 20 wt % barium produced by concentrating the filtered product by vacuum distillation had a basicity of 2.

## Examples 1 to 4

Preparation of basic barium carboxylates according to the invention.

The procedure indicated in Comparative Example A was repeated, with the omission of the step of concentrating the filtered product, replacing the neodecanoic acid by the acid or acid mixture indicated in Table II below. In each case a product with a basicity of 2.5 and a barium content of 20 wt % was obtained directly, without the need for the concentration step used in Comparative Examples A and B. In the Table, amounts are given in grams.

TABLE II

|  | Ex.1 | Ex.2 | Ex.3 | Ex.4 |
|---|---|---|---|---|
| $Ba(OH)_2.8H_2O$ | 459 | 459 | 459 | 459 |
| Cekanoic C8 acid | 168 | - | - | - |
| Cekanoic C7 acid | - | 151 | - | - |
| Cekanoic C9 acid | - | - | 184 | - |
| Neooctanoic acid | - | - | - | 168 |
| $CO_2$ | 38 | 38 | 38 | 38 |
| Dearomatized white spirit | 543 | 561 | 527 | 543 |
| Triethylene glycol | 50 | 50 | 50 | 50 |
| Water | 500 | 500 | 500 | 500 |

## Example 5

The effect of the product of Example 1 as a stabiliser for polyvinyl chloride was compared with the commercially available overbased barium nonyl phenolate at the same barium level in the formulation. The stabiliser formulation, the polyvinyl chloride formulation and the heat stability data are given in the following TABLE III.

6

## TABLE III

| STABILISER COMPOSITION | CONTENT wt% | |
|---|---|---|
| Component | Formulation 1 | Formulation 2 |
| Solution of mixed barium and zinc salts of oleic and p-tert butyl benzoic acids | 57 | 57 |
| Overbased barium isooctanoate (Cekanoic C8 acid) (20 wt% barium) | - | 20 |
| Overbased barium nonyl phenolate (28 wt% barium) | 14.3 | - |
| Diphenyl isooctylphosphite | 20 | 20 |
| Diketone costabilizer | 3 | 3 |
| White Spirit | 5.7 | - |

Polyvinyl Chloride Formulation
Solvic 246GA          100  parts
Dioctyl phthalate      25  parts
Expoxidised soya bean oil   1.5 parts
Stabilizer             2.5 parts

Heat Stability Performance

Oven Test at 190°C

| | Formulation 1 | Formulation 2 |
|---|---|---|
| A.   Initial colour | +++ | +++ |
| B.   Colour hold (40-50 mins) | ++ | ++ |
| C.   Complete loss of colour | 90 mins | 90 mins |

Dynamic Test (two roll mill) at 180°C

| | | |
|---|---|---|
| D.   Initial colour | +++ | +++ |
| E.   Colour hold (20-30 mins) | ++ | +++ |
| F.   Colour hold (40-45 mins) | + | ++ |

+++ = Excellent, ++ = Good, + = Poor

The test results show that use of the basic barium salts of acids of this invention give significantly better dynamic colour control in comparison with currently used barium salts.

The Oven Test is performed by cutting a roll milled sheet of the polyvinyl chloride into small pieces which are placed in the oven at the indicated temperature on glass plates and after the indicated time are compared with a standard reference.

In the Organic Test the made up compound is processed on a two roll whose rolls are at the indicated temperature for the time indicated and the colour compared with a standard reference.

**Claims**

1. A basic barium carboxylate wherein at least 85 weight % of the carboxylate groups consist of groups derivable from at least one branched chain aliphatic monocarboxylic acid having 6 to 9 carbon atoms.

2. A barium carboxylate as claimed in claim 1, wherein in at least one acid from which the carboxylate groups are derivable the or each branching group is a methyl or ethyl group.

3. A barium carboxylate as claimed in claim 1 or 2, wherein at least one acid from which the carboxylate groups are derivable there is at least one branching group on a carbon atom other than the α-carbon atom.

4. A barium carboxylate as claimed in claim 3, wherein the said one acid also has at least one methyl group on the α-carbon atom.

5. A barium carboxylate as claimed in claim 1, wherein the carboxylate groups consist essentially of groups derivable from a mixture of acids each having 8 carbon atoms and being branched predominantly in non-α positions.

6. A barium carboxylate as claimed in any one of claims 1 to 5, which has a basicity of at least 2.

7. A barium carboxylate as claimed in any one of claims 1 to 6, in the form of a colloidal solution in an inert organic liquid, the colloidal solution having a barium content of at least 20 wt%.

8. A barium carboxylate as claimed in any one of claims 1 to 7, in the form of a complex which also contains zinc.

9. A process for preparing a basic barium carboxylate as claimed in claim 1, which comprises reacting a basic barium compound with an acidic gas in an organic liquid in the presence of (a) at least one monocarboxylic acid as specified in claim 1 and a barium compound capable of reacting with the said acid and/or (b) a barium carboxylate obtainable by reaction of the acid(s) and barium compound specified in (a).

10. A process as claimed in claim 9, wherein the same barium compound is used for reaction with the monocarboxylic acid(s) as is used for reaction with the acidic gas.

11. A process as claimed in claim 9 or claim 10, wherein reaction of the barium compound with the carboxylic acid(s) is carried out in the presence of sufficient water to dissolve the barium compound.

12. A process as claimed in any one of claims 9 to 11, wherein substantially all the water present in the reaction medium is removed before introduction of the acidic gas.

13. A process as claimed in any one of claims 9 to 20, wherein the acidic gas is carbon dioxide.

14. A process as claimed in any one of claims 9 to 13, wherein the reaction mixture also comprises a zinc compound capable of reacting with the monocarboxylic acid(s).

15. A polymer composition comprising a halogen-containing polymer and a barium carboxylate as claimed in any one of claims 1 to 8.

16. The use of a basic barium carboxylate as claimed in any one of claims 1 to 8 for stabilizing a halogen-containing polymer.

17. A composition or use as claimed in claim 15 or claim 16, wherein the polymer is a homo- or copolymer of vinyl chloride.

18. A composition or use as claimed in any one of claims 15 to 17, wherein the proportion of barium is from 0.025 to 0.25 wt%, based on the weight of the polymer and any plasticizer that may be present.

**Patentansprüche**

1. Basisches Bariumcarboxylat, bei dem mindestens 85 Gew.% der Carboxylatgruppen aus Gruppen zusammengesetzt sind, die aus mindestens einer verzweigtkettigen aliphatischen Monocarbonsäure mit 6 bis 9 Kohlenstoffatomen ableitbar sind.

2. Bariumcarboxylat nach Anspruch 1, bei dem in mindestens einer Säure, aus der sich die Carboxylatgruppen ableiten lassen, die Verzweigungsgruppe oder jede Verzweigungsgruppe eine Methyl- oder Ethylgruppe ist.

3. Bariumcarboxylat nach Anspruch 1 oder 2, bei dem mindestens eine Säure, aus der sich die Carboxylatgruppen ableiten lassen, mindestens eine Verzweigungsgruppe an einem anderen Kohlen-stoffatom als dem $\alpha$-Kohlenstoffatom aufweist.

4. Bariumcarboxylat nach Anspruch 3, bei dem diese eine Säure außerdem mindestens eine Methylgrup-pe am $\alpha$-Kohlenstoffatom aufweist.

5. Bariumcarboxylat nach Anspruch 1, bei dem die Carboxylatgruppen im wesentlichen aus Gruppen zusammengesetzt sind, die sich aus einer Mischung von Säuren ableiten lassen, die jeweils 8 Kohlenstoffatome aufweisen und vorwiegend in anderen als der $\alpha$-Stellung verzweigt sind.

6. Bariumcarboxylat nach einem der Ansprüche 1 bis 5, das eine Basizität von mindestens 2 aufweist.

7. Bariumcarboxylat nach einem der Ansprüche 1 bis 6 in Form einer kolloidalen Lösung in einer inerten organischen Flüssigkeit, wobei die kolloidale Lösung einen Bariumgehalt von mindestens 20 Gew.% aufweist.

8. Bariumcarboxylat nach einem der Ansprüche 1 bis 7 in Form eines Komplexes, der auch Zink enthält.

9. Verfahren zur Herstellung von basischem Bariumcarboxylat gemäß Anspruch 1, bei dem eine basische Bariumverbindung mit einem sauren Gas in einer organischen Flüssigkeit in Gegenwart von (a) mindestens einer Monocarbonsäure wie in Anspruch 1 spezifiziert und einer Bariumverbindung, die zur Umsetzung mit dieser Säure in der Lage ist, und/oder (b) einem Bariumcarboxylat, das durch die Umsetzung der in (a) angegebenen Säure(n) und Bariumverbindung erhältlich ist, umgesetzt wird.

10. Verfahren nach Anspruch 9, bei dem die gleiche Bariumverbindung für die Umsetzung mit der Monocarbonsäure bzw. den Monocarbonsäuren wie für die Umsetzung mit dem sauren Gas verwendet wird.

11. Verfahren nach Anspruch 9 oder 10, bei dem die Umsetzung der Bariumverbindung mit der Carbonsäure bzw. den Carbonsäuren in Gegenwart von ausreichend Wasser durchgeführt wird, um die Bariumverbindung aufzulösen.

12. Verfahren nach einem der Ansprüche 9 bis 11, bei dem im wesentlichen das gesamte in dem Reaktionsmedium vorhandene Wasser vor Einbringung des sauren Gases entfernt wird.

13. Verfahren nach einem der Ansprüche 9 bis 12, bei dem das saure Gas Kohlendioxid ist.

14. Verfahren nach einem der Ansprüche 9 bis 13, bei dem die Reaktionsmischung auch eine Zinkverbindung umfaßt, die zur Umsetzung mit der Monocarbonsäure bzw. den Monocarbonsäuren in der Lage ist.

15. Polymerzusammensetzung, die ein halogenhaltiges Polymer und ein Bariumcarboxylat gemäß einem der Ansprüche 1 bis 8 umfaßt.

16. Verwendung eines basischen Bariumcarboxylats nach einem der Ansprüche 1 bis 8 zum Stabilisieren von halogenhaltigem Polymer.

17. Zusammensetzung oder Verwendung nach Anspruch 15 oder Anspruch 16, bei dem das Polymer ein Homo- oder Copolymer aus Vinylchlorid ist.

18. Zusammensetzung oder Verwendung nach einem der Ansprüche 15 bis 17, bei dem der Bariumanteil 0,025 bis 0,25 Gew.% beträgt, bezogen auf das Gewicht des Polymers und beliebigem möglicherweise vorhandenen Weichmacher.

**Revendications**

1. Carboxylate de baryum basique dans lequel au moins 85 % en poids des groupes carboxylate consistent en groupes pouvant être issus d'au moins un acide monocarboxylique aliphatique à chaîne ramifiée, ayant 6 à 9 atomes de carbone.

2. Carboxylate de baryum suivant la revendication 1, dans lequel, dans au moins un acide à partir duquel peuvent être issus les groupes carboxylate, le ou chaque groupe de ramification est un groupe méthyle ou éthyle.

3. Carboxylate de baryum suivant la revendication 1 ou 2, dans lequel, dans au moins un acide duquel les groupes carboxylate peuvent être issus, il existe au moins un groupe de ramification sur un atome de carbone autre que l'atome de carbone en position α.

4. Carboxylate de baryum suivant la revendication 3, dans lequel l'acide possède également au moins un groupe méthyle sur l'atome de carbone en position α.

5. Carboxylate de baryum suivant la revendication I, dans lequel les groupes carboxylate consistent essentiellement en groupes pouvant être issus d'un mélange d'acides ayant chacun 8 atomes de carbone et étant ramifiés principalement dans des positions autres que la position α.

6. Carboxylate de baryum suivant l'une quelconque des revendications 1 à 5, qui possède une basicité d'au moins 2.

7. Carboxylate de baryum suivant l'une quelconque des revendications 1 à 6, sous forme d'une solution colloïdale dans un liquide organique inerte, la solution colloïdale ayant une teneur en baryum d'au moins 20 % en poids.

8. Carboxylate de baryum suivant l'une quelconque des revendications 1 à 7, sous forme d'un complexe qui contient également du zinc.

9. Procédé de préparation d'un carboxylate de baryum basique suivant la revendication 1, qui comprend la réaction d'un composé de baryum basique avec un gaz acide dans un liquide organique en présence (a) d'au moins un acide monocarboxylique tel que spécifié dans la revendication 1 et d'un composé de baryum capable de réagir avec cet acide et/ou (b) d'un carboxylate de baryum pouvant être obtenu par réaction du ou des acides et du composé de baryum spécifiés en (a).

10. Procédé suivant la revendication 9, dans lequel le même composé de baryum est utilisé pour la réaction avec le ou les acide(s) monocarboxylique(s) et pour la réaction avec le gaz acide.

11. Procédé suivant la revendication 9 ou la revendication 10, dans lequel la réaction du composé de baryum avec le ou les acide(s) carboxylique(s) est conduite en présence d'une quantité d'eau suffisante pour dissoudre le composé de baryum.

12. Procédé suivant l'une quelconque des revendications 9 à 11, dans lequel pratiquement la totalité de l'eau présente dans le milieu réactionnel est éliminée avant introduction du gaz acide.

13. Procédé suivant l'une quelconque des revendications 9 à 20, dans lequel le gaz acide est l'anhydride carbonique.

14. Procédé suivant l'une quelconque des revendications 9 à 13, dans lequel le mélange réactionnel comprend également un composé de zinc capable de réagir avec le ou les acide(s) monocarboxylique-(s).

15. Composition polymérique comprenant un polymère halogéné et un carboxylate de baryum suivant l'une quelconque des revendications 1 à 8.

16. Utilisation d'un carboxylate de baryum basique suivant l'une quelconque des revendications 1 à 8 pour la stabilisation d'un polymère halogéné.

17. Composition ou utilisation suivant la revendication 15 ou la revendication 16, dans laquelle le polymère est un homo- ou copolymère de chlorure de vinyle.

18. Composition ou utilisation suivant l'une quelconque des revendications 15 à 17, dans laquelle la proportion de baryum est comprise dans l'intervalle de 0,025 à 0,25 % en poids, sur la base du poids du polymère et de n'importe quel plastifiant pouvant être présent.